# EUROPEAN PATENT APPLICATION

(11) **EP 4 230 226 A1**
(43) Date of publication of application: **23.08.2023**
(21) Application number: 21880496.1
(22) Date of filing: 13.10.2021
(51) Int. Cl.: A61K 48/00, A61K 31/713, A61K 9/00, A61P 27/02

(54) **COMPOSITION FOR TREATING RETINAL OR CHOROIDAL DISEASES, CONTAINING ACTA2 INHIBITOR AS ACTIVE INGREDIENT**

(30) Priority: 13.10.2020 KR 20200131954; 12.10.2021 KR 20210134901
(71) Applicant: The Asan Foundation, Seoul 05505 (KR); University Of Ulsan Foundation For Industry Cooperation, Ulsan 44610 (KR)
(72) Inventor: LEE, Junyeop, Seoul 05555 (KR); KIM, Soo Jin, Daegu 42471 (KR)
(74) Representative: Dehns
(86) International application number: PCT/KR2021/014092
(87) International publication number: WO 2022/080849

(57) **Abstract**

The present invention relates to: a composition for treating retinal or choroidal diseases, comprising an ACTA2 inhibitor as an active ingredient; and the like. The present inventors have confirmed that, since an increase in ACTA2 induces a decrease in perivascular cells and induces the proliferation and distribution of smooth muscle cells, the proliferation and distribution of vascular smooth cells are inhibited, the distribution of perivascular cells is increased, and the dilation and leakage of choroid vessels are inhibited, as a result of administration of ACTA2 inhibitors siRNA and miRNA to an animal model having a retinal or choroidal disease. Therefore, an ACTA2 inhibitor is expected to be effectively used in the treatment of retinal or choroidal diseases.

## Description

### [Technical Field]

The present invention relates to a composition for treating retinal or choroidal diseases, comprising an ACTA2 inhibitor as an active ingredient, and the like.

The present application claims priority to and the benefit of Korean Patent Application Nos. 10-2020-0131954 and 10-2020-0134901 filed in the Korean Intellectual Property Office on October 13, 2020 and October 12, 2021, respectively, and all the contents disclosed in the specification and drawings of these applications are incorporated in the present application.

### [Background Art]

The nerve tissue located in the center of the inner retina of the eye is called the macula, and since most of the visual cells responding to a light stimulus are gathered in the macula and a place where an image of an object is formed is also at the center of the macula, the macula plays a very important role in vision. Age-related macular degeneration (AMD) is a chronic disease characterized by degeneration of the retinal pigment epithelium, Bruch's membrane, and choroidal capillaries of the macula. Anatomically, the neurosensory retina is located in front of the retinal pigment epithelium and depends on the retinal pigment epithelium for its nutrition, support, recirculation, and waste disposal. The Bruch's membrane is a five-layered structure, and is interposed between the choroid and the retinal pigment epithelium. The innermost layer is the basement membrane of the retinal pigment epithelium and the outermost layer is the basement membrane of the choroidal capillary endothelial cells. That is, age-related macular degeneration is a degenerative disease that develops in the retinal pigment epithelium, Bruch's membrane and choroidal capillary complex.

This disease mainly occurs in people aged 50 years or older, and in the West, this disease is already the main cause of blindness in people aged 60 years or older, and it also tends to be increasing in Korea. Although the cause of AMD is not clearly understood yet, there are age (which exhibits a sharp increase particularly after the age of 75), hypertension, obesity, genetic predisposition, excessive UV exposure, low serum antioxidant concentration, and the like, in addition to smoking which is an environmental factor that have received the most attention, as the known risk factors.

There are two types of macular degeneration, which are dry (nonexudative) macular degeneration and wet (exudative) macular degeneration. In the case of dry macular degeneration (dry AMD, nonexudative AMD, and nonneovascular AMD), waste products form a yellow deposit called drusen under the retina and when this deposit accumulates, it disturbs blood flow to the retina particularly to the macula, resulting in the blurry vision and visual impairment. Dry macular degeneration does not cause rapid vision loss, but may progress to wet macular degeneration. Wet macular degeneration (wet AMD, exudative AMD, neovascular AMD) results from the growth of new blood vessels in the choroidal portion beneath the retina. When the weak new blood vessel is broken, it results in bleeding or exudation, which causes degeneration of the macular region of the retina to cause vision impairment. It is known that wet macular degeneration progresses rapidly, leading to rapid deterioration of vision within several weeks and blindness within two months to three years.

Meanwhile, as known treatment methods for macular degeneration to date, photodynamic therapy (PDT) and anti-vascular endothelial growth factor antibody (anti-VEGF) injection have been used. PDT is a method of selectively destroying new blood vessels by irradiating the eyes with a special laser only reacting to a light-sensitive material when visudyne, which is the light-sensitive material, is injected through a blood vessel and then reaches the new blood vessels of the retina after a predetermined time. However, there are many cases of recurrence even after treatment, so that there are disadvantages in that the treatment must be repeated in many cases, and the retina itself may also be damaged.

Anti-VEGF injection is a method (intravitreal injection) of directly injecting an antibody (anti-VEGF) that inhibits the formation and proliferation of new blood vessels by selectively binding to vascular endothelial growth factor (VEGF), which is an important factor for the generation and progression of new blood vessels. Examples of the protein antibodies used for anti-VEGF injection include Lucentis and Avastin, Lucentis is a drug approved by the FDA as a therapeutic agent for wet macular degeneration, and Avastin is a drug approved for the treatment of cancer and is used for eye diseases. However, the protein antibody injection treatment method is expensive, and a drug is directly injected into the eye because the drug cannot be dropped or applied to the eye, and needs to be periodically administered about once every month, so there is a risk of bleeding, pain, infection, retinal detachment, and the like.

Age-related macular degeneration, diabetic retinopathy, and diabetic choroidopathy are commonly caused by structural and functional abnormalities of intraocular choroidal blood vessels. Although the most important cells for maintaining vascular structure and function are pericytes, changes in choroidal pericytes in aged and diabetic states have not been studied to date. Pericytes surround vascular endothelial cells, and are the most important cells for maintaining the structure and function of blood vessels. Although the intraocular injection of an anti-vascular endothelial growth factor (VEGF) antibody has been currently used as a therapeutic agent for the two diseases to suppress angiogenesis and vascular leakage in choroidal vessels, it is presumed that the VEGF antibody does not affect the distribution and function of the pericytes of the choroidal vessels, which are important cells for vessel stability.

Therefore, in order to maintain the structure and function of stable choroidal vessels, it is important to maintain the normal distribution and function of pericytes, and there is a need for developing a new technique which treats the diseases while maintaining the ultra-microstructure and function of these choroidal vessels.

### [Disclosure]

### [Technical Problem]

Thus, the present inventors confirmed that ACTA2 inhibitors induce normalization of pericytes of choroidal vessels for the treatment of retinal diseases including age-related macular degeneration and diabetic chorioretinopathy, thereby completing the present invention.

Accordingly, an object of the present invention is to provide a pharmaceutical composition for preventing or treating retinal or choroidal diseases, comprising an ACTA2 inhibitor as an active ingredient.

Another object of the present invention is to provide a quasi-drug composition for preventing or treating retinal or choroidal diseases, comprising an ACTA2 inhibitor as an active ingredient.

However, the technical problems to be achieved by the present invention are not limited to the aforementioned problems, and other problems that are not mentioned may be clearly understood by those skilled in the art from the following description.

### [Technical Solution]

In order to solve the above problems, the present invention provides a pharmaceutical composition for preventing or treating retinal or choroidal diseases, comprising an ACTA2 inhibitor as an active ingredient.

In an exemplary embodiment of the present invention, the inhibitor may be an ACTA2 activity inhibitor or an expression inhibitor, but is not limited thereto.

In another exemplary embodiment of the present invention, the activity inhibitor may be one or more selected from the group consisting of a compound, a peptide, a peptidomimetic, a substrate analogue, an aptamer and an antibody, which specifically bind to an ACTA2 protein, but is not limited thereto.

In still another exemplary embodiment of the present invention, the expression inhibitor may be one or more selected from the group consisting of an antisense nucleotide, RNAi, siRNA, miRNA, shRNA, and a ribozyme, which complementarily bind to the mRNA of an ACTA2 gene, but is not limited thereto.

In yet another exemplary embodiment of the present invention, the siRNA may include one or more base sequences selected from the group consisting of SEQ ID NOS: 3 to 8, but is not limited thereto.

In yet another exemplary embodiment of the present invention, the siRNA may include one or more siRNAs selected from the group consisting of SEQ ID NOS: 3 and 4; SEQ ID NOS: 5 and 6; and SEQ ID NOS: 7 and 8, but is not limited thereto.

In yet another exemplary embodiment of the present invention, the miRNA may be miR-4524a, but is not limited thereto.

In yet another exemplary embodiment of the present invention, the retinal or choroidal disease may be one or more selected from the group consisting of retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular capillarectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration due to glaucoma, and ischemic optic neuropathy, but is not limited thereto.

In yet another exemplary embodiment of the present invention, the ACTA2 protein may include an amino acid sequence represented by SEQ ID NO: 1, but is not limited thereto.

In yet another exemplary embodiment of the present invention, the composition may be administered to a subject in need thereof by one or more routes selected from the group consisting of oral, subcutaneous, intraperitoneal, intrapulmonary, intranasal, intramuscular, intravenous, intraarterial and topical ocular administration, but is not limited thereto.

In yet another exemplary embodiment of the present invention, the topical ocular administration may be one selected from the group consisting of intraconjunctival administration, intravitreal administration, subretinal administration, suprachoroidal administration, subconjunctival administration, and sub-Tenon's capsule administration, but is not limited thereto.

In yet another exemplary embodiment of the present invention, the composition may further include an anti-VEGF agent, but is not limited thereto.

In yet another exemplary embodiment of the present invention, the pharmaceutical composition may be administered simultaneously or sequentially with the anti-VEGF agent, but is not limited thereto.

In yet another exemplary embodiment of the present invention, the composition may have one or more effects selected from the group consisting of the following items, but is not limited thereto.
inhibition of the proliferation or distribution of smooth muscle cells;
promotion of the proliferation or distribution of pericytes;
drusen reduction;
inhibition of retinal neovascularization or vascular leakage; and
inhibition of the dilation or leakage of choroidal vessels.

Further, the present invention provides a quasi-drug composition for preventing or treating retinal or choroidal diseases, comprising an ACTA2 inhibitor as an active ingredient.

In addition, the present invention provides a method for preventing, ameliorating, or treating retinal or choroidal diseases, the method including administering a composition comprising an ACTA2 inhibitor as an active ingredient to a subject in need.

Furthermore, the present invention provides a use of a composition comprising an ACTA2 inhibitor as an active ingredient for preventing, ameliorating, or treating retinal or choroidal diseases.

Further, the present invention provides a use of an ACTA2 inhibitor for producing a drug for preventing or treating retinal or choroidal diseases.

### [Advantageous Effects]

Based on the fact that an increase in ACTA2 induces a decrease in pericytes and induces the proliferation and distribution of smooth muscle cells, the present inventors have confirmed that the proliferation and distribution of vascular smooth cells are inhibited, the distribution of pericytes is increased, and the dilation and leakage of choroid vessels are inhibited, as a result of administration of ACTA2 inhibitor siRNA and miRNA to an animal model having a retinal or choroidal disease. Therefore, an ACTA2 inhibitor is expected to be effectively used in the treatment of retinal or choroidal diseases.

### [Description of Drawings]

FIG. 1 illustrates the results of observing smooth muscle cells and pericytes in the choroidal capillary layer according to the age of a person according to an exemplary embodiment of the present invention.
FIG. 2 illustrates the expression patterns of smooth muscle cell-related genes and pericyte-related genes in the choroidal capillary layer according to the age of a person according to an exemplary embodiment of the present invention.
FIG. 3 illustrates the ACTA2 expression level as a result of treating mouse choroid tissue with ACTA2 siRNA according to an exemplary embodiment of the present invention.
FIG. 4 illustrates the expression levels of PDGFRβ, which is a pericyte marker, and TAGLN (Transgelin), which is a smooth muscle cell marker, as a result of injecting ACTA2 siRNA into the vitreous body of aged mice according to an exemplary embodiment of the present invention.
FIG. 5 confirms smooth muscle cells in the retina and choroid as a result of injecting ACTA2 siRNA and miRNA into the vitreous body of aged mice according to an exemplary embodiment of the present invention.
FIG. 6 illustrates drusen-suppressing effects through injection of ACTA2 siRNA and miRNA into the vitreous body of a dry age-related macular degeneration (dAMD) mouse model according to an exemplary embodiment of the present invention.
FIG. 7 illustrates the results of confirming the injection of ACTA2 siRNA and miRNA into the vitreous body of a dry age-related macular degeneration (dAMD) mouse model according to an exemplary embodiment of the present invention by an electroretinogram.
FIG. 8 illustrates the distributions of choroidal neovascularization, pericytes and smooth muscle cells as a result of injection of ACTA2 siRNA and miRNA into the vitreous body of a wet age-related macular degeneration (wAMD) mouse model according to an exemplary embodiment of the present invention.
FIG. 9 illustrates the results of fluorescein angiography (FAG) and indocyanine-green angiography (ICGA) for confirming neovascularization and vascular leakage after injection of ACTA2 siRNA and miRNA into the vitreous body of a diabetic mouse model according to an exemplary embodiment of the present invention.
FIG. 10 illustrates the effect of suppressing the microaneurysms of the retina as a result of injecting ACTA2 siRNA and miRNA into the vitreous body of a diabetic mouse model according to an exemplary embodiment.
FIG. 11 illustrates the effect of reducing the dilation and leakage of choroidal vessels as a result of injecting ACTA2 siRNA and miRNA into the vitreous body of a diabetic mouse model according to an exemplary embodiment of the present invention.
FIG. 12 illustrates the effect of alleviating choroidal inflammation through confirmation of aggregation of microglia as a result of injecting ACTA2 siRNA and miRNA into the vitreous body of a diabetic mouse model according to an exemplary embodiment of the present invention.

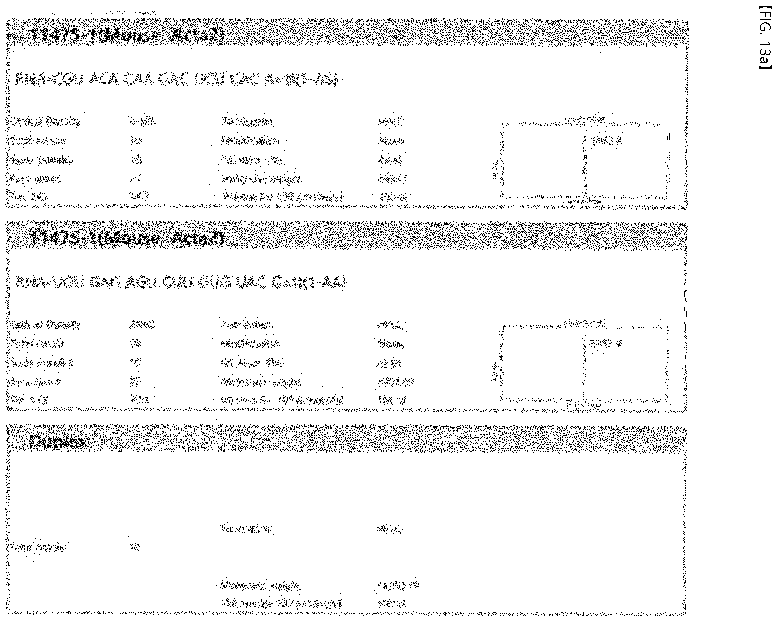

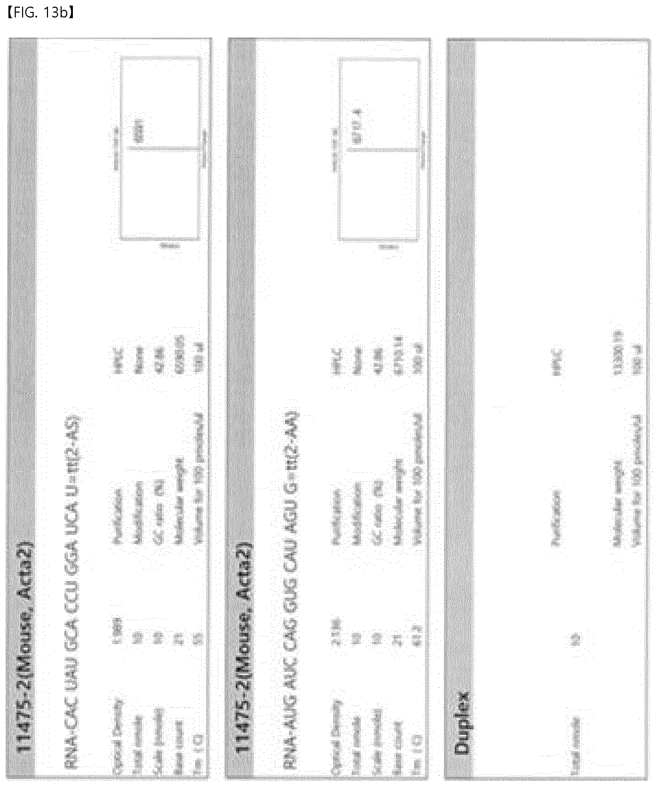

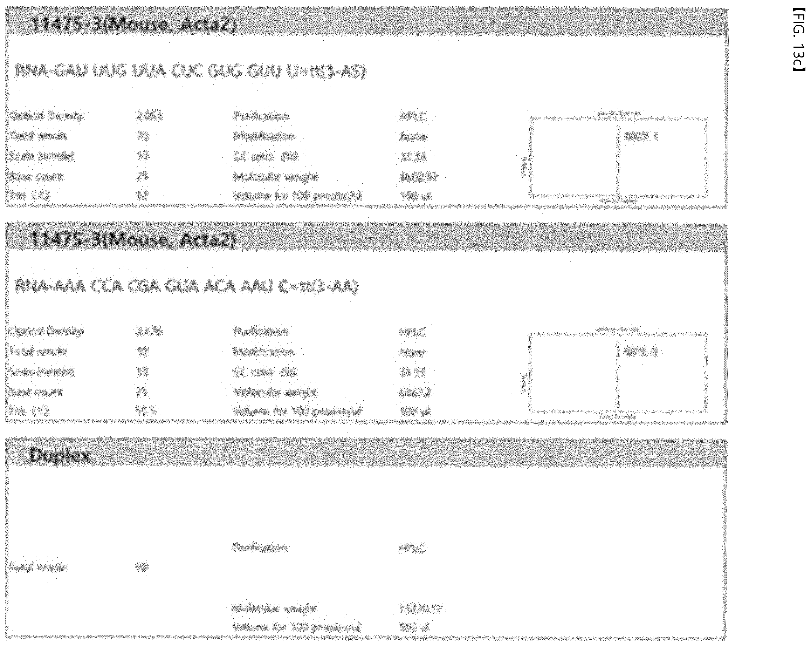

FIGS. 13A to 13C illustrate information on ACTA2 siRNA #1 to #3 used according to an exemplary embodiment of the present invention.

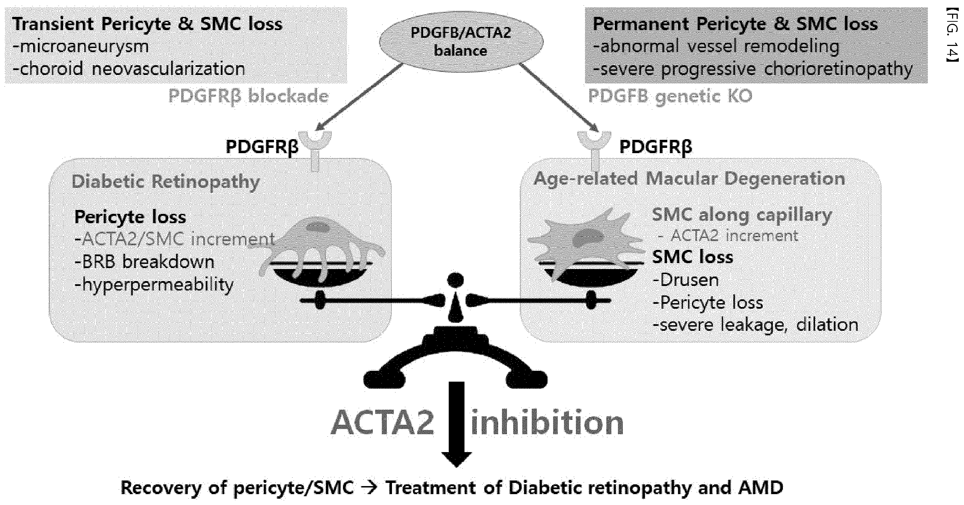

FIG. 14 illustrates the effect mechanism of ameliorating retinal degeneration through ACTA2 inhibition.

### [Modes of the Invention]

Through exemplary embodiments, the present inventors, on the basis that smooth muscle cells increase in choroidal capillaries in diabetic retinopathy mouse models and age-related macular degeneration (AMD) mouse models,
confirmed that by administering an ACTA2 inhibitor to the vitreous body, the excessive proliferation and distribution of smooth muscle cells in the choroidal vessels of aged mice was inhibited, and the distribution of pericytes was increased (see Example 4),
confirmed that by administering an ACTA2 inhibitor to the vitreous body of an AMD animal model, the generation of drusen was effectively inhibited and the function of visual cells was also restored (see Examples 5 and 6),
confirmed that by administering an ACTA2 inhibitor to the vitreous body of a wet AMD animal model, the size of choroidal neovascularization was markedly decreased and the distribution of pericytes was significantly increased while the distribution of smooth muscle cells was also significantly decreased (see Example 7), and
confirmed that by administering an ACTA2 inhibitor to the vitreous body of a diabetic animal model, choroidal neovascularization and vascular leakage were reduced, retinal microvascular production was reduced, the dilation and leakage of choroidal vessels were reduced, and choroidal inflammation was alleviated (see Example 8), thereby completing the present invention.

Accordingly, the present invention relates to a pharmaceutical composition for preventing or treating retinal or choroidal diseases, comprising an ACTA2 inhibitor as an active ingredient.

Hereinafter, the present invention will be described in detail.

As used herein, the term "protein" is interchangeably with 'polypeptide' or 'peptide,' and refers to, for example, a polymer of amino acid residues as generally found in proteins in a natural state.

As used herein, "polynucleotide" or "nucleic acid" refers to deoxyribonucleic acid (DNA) or ribonucleic acid (RNA) in either a single- or double-stranded form. Unless otherwise limited, the term also encompasses known analogs of natural nucleotides which are hybridized to a nucleic acid in a manner similar to naturally occurring nucleotides. In general, DNA consists of four bases such as adenine (A), guanine (G), cytosine (C), and thymine (T), and RNA has uracil (U) instead of thymine. In a nucleic acid double strand, A forms a hydrogen bond with a T or U base, and C forms a hydrogen bond with a G base, and such a base relationship is called "complementary."

Meanwhile, 'messenger RNA (mRNA)' is an RNA which serves as a blueprint for polypeptide synthesis (protein translation) by transmitting genetic information of the base sequence of a specific gene to ribosomes in the process of protein synthesis. Using a gene as a template, single-stranded mRNA is synthesized through the process of transcription.

As used herein, "actin alpha 2, smooth muscle (ACTA2)" is also called smooth muscle actin, SMA, a-SMA, AAT6 or ACTSA, and refers to a gene located on chromosome 10 (#chr10:90,682,710-90,735,753(53,043)), and the specific base sequence and protein information thereof are publicly known in the NCBI (NCBI Reference Sequence: NP_001135417.1, NP_001307784.1, or NP_001604.1).

In the present invention, the ACTA2 protein may include or consist of an amino acid sequence of SEQ ID NO: 1, but is not limited thereto.

In the present invention, a gene encoding the ACTA2 protein may include a base sequence represented by SEQ ID NO: 2, but is not limited thereto. As used herein, the "gene encoding the ACTA2 protein" may include a gene base sequence represented by SEQ ID NO: 2, and preferably may consist of a base sequence represented by SEQ ID NO: 2. Further, the gene variant is included in the scope of the present invention. Specifically, the gene may include a base sequence having a sequence homology of 70 % or more, more preferably 80 % or more, and most preferably 90 % or more to the base sequence of SEQ ID NO: 2. For example, the gene includes a polypeptide having a sequence homology of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

As used herein, the term "complementary" means that under predetermined hybridization or annealing conditions, specifically physiological conditions (intracellular), a target moiety in a nucleic acid molecule is sufficiently complementary to be selectively hybridized to a target (for example, an ACTA2 gene), and means that the intranucleic acid targeting moiety may have one or more mismatched base sequences, both substantially complementary and perfectly complementary are encompassed, and more specifically, the intranucleic acid targeting moiety is perfectly complementary.

In an exemplary embodiment of the present invention, the inhibitor may be an ACTA2 activity inhibitor or an expression inhibitor, but is not limited thereto.

In the present specification, the activity inhibitor may be one or more selected from the group consisting of a compound, a peptide, a peptidomimetic, a substrate analogue, an aptamer and an antibody, which specifically bind to an ACTA2 protein, but is not limited thereto.

In the present specification, the expression inhibitor may be one or more selected from the group consisting of an antisense nucleotide, RNAi, siRNA, miRNA, shRNA, and a ribozyme, which complementarily bind to the mRNA of an ACTA2 gene, but is not limited thereto.

As used herein, the term "siRNA" means that a sense strand (for example, a sequence corresponding to an ACTA2 gene mRNA sequence) and an antisense strand (for example, a sequence complementary to the ACTA2 gene mRNA sequence) are located opposite each other, and thus, may have a double-stranded structure. In addition, the siRNA molecule that can be used in the present invention may have a single-stranded structure having self-complementary sense and antisense strands. siRNA is not limited to complete pairing of double-stranded RNA moieties in which RNA pairs, but may include moieties that do not form a pair due to mismatches (corresponding bases are not complementary), bulges (no corresponding bases on one strand), and the like. Specifically, the total length is 10 to 100 bases, more specifically 15 to 80 bases, and more specifically 20 to 70 bases. In the present invention, the siRNA may include one or more base sequences selected from the group consisting of SEQ ID NOS: 3 to 8, but is not limited thereto. Furthermore, in the present invention, the siRNA may include one or more siRNAs selected from the group consisting of SEQ ID NOS: 3 and 4 (siRNA #1); SEQ ID NOS: 5 and 6 (siRNA #2); and SEQ ID NOS: 7 and 8 (siRNA #3), but is not limited thereto.

In the present specification, the siRNA #1, #2, and #3 may be included at a molar ratio of 1 to 10 : 1 to 10 : 1 to 10, 1 to 5 : 1 to 5 : 1 to 5, 1 to 3 : 1 to 3 : 1 to 3, 1 to 2 : 1 to 2 : 1 to 2, or 1 : 1 : 1, but are not limited thereto.

As used herein, the term "small hairpin RNA or short hairpin RNA (shRNA)" refers to a sequence of RNA that makes a tight hairpin turn that can be used to silence gene expression via RNA interference. The shRNA may be introduced into cells using any promoter capable of functioning in eukaryotic cells. The shRNA hairpin structure is degraded into siRNA that is intracellular machinery, and bound to an RNA-induced silencing complex. The above-described complex binds to and degrades mRNA matched to the siRNA bound thereto. The shRNA is transcribed by RNA polymerase III, and shRNA production in mammalian cells may trigger interferon responses, just as cells recognize shRNA as viral attacks and seek defenses.

As used herein, the term "microRNA (miRNA)" refers to a material that controls gene expression in eukaryotes by binding to the 3'-UTR of messenger RNA (mRNA) as a single-stranded RNA molecule of 21 to 25 nucleotides (BartelDP, et al., Cell, 23;116(2): 281-297(2004)). The production of miRNA is made into a precursor miRNA (pre-miRNA) of a stem-loop structure by Drosha (RNaseIII type enzyme), moves to the cytoplasm, and is cleaved by Dicer to make mature miRNA. In the present invention, the miRNA may be miR-4524a, but is not limited thereto. The miR-4524a may include or consist of SEQ ID NO: 9, but is not limited thereto.

As used herein, the term "antisense oligonucleotide" refers to DNA or RNA comprising a nucleic acid sequence complementary to that of a specific mRNA, or derivatives thereof, and may bind to a complementary sequence within mRNA to inhibit the translation of mRNA into a protein. For example, the antisense sequence of the present invention refers to a DNA or RNA sequence that is complementary to ACTA2 and capable of binding to ACTA2 mRNA, and such an antisense sequence may inhibit activity that is essential for the translation, translocation into a cytoplasm, or maturation of the ACTA2 mRNA, or for all other overall biological functions. The length of the antisense nucleic acid is 6 to 100 bases, specifically 8 to 60 bases, and more specifically 10 to 40 bases.

As used herein, the term "ribozyme" refers to an RNA having the same function as an enzyme that recognizes the nucleotide sequence of a specific RNA and autonomously cleaves it as a type of RNA. A ribozyme is a complementary base sequence of a target messenger RNA strand, consisting of a region that binds with specificity and a region that cleaves the target RNA. As used herein, the term "aptamer" refers to an oligonucleotide (generally, an RNA molecule) that binds to a specific target. Specifically, "aptamer" as used herein refers to an oligonucleotide aptamer (for example, an RNA aptamer).

In the present specification, siRNA or shRNA may include various modifications for improving the *in vivo* stability of oligonucleotides, imparting resistance to nucleolytic enzymes, and reducing non-specific immune reactions. For the modification of the oligonucleotides, it is possible to use a combination of one or more modifications selected from the modification by substitution of an OH group at the 2' carbon position of a sugar structure in one or more nucleotides with - CH₃ (methyl), -OCH₃ (methoxy), -NH₂, -F, -O-2-methoxyethyl, -O-propyl, -O-2-methylthioethyl, -O-3-aminopropyl, -O-3-dimethylaminopropyl, -O-N-methylacetamido or -O-dimethylamidooxyethyl; modification in which oxygen in a sugar structure in nucleotides is substituted with sulfur; and modification of nucleotide linkages to phosphorothioate, boranophosphate, or methyl phosphonate linkages, and modification to a peptide nucleic acid (PNA), locked nucleic acid (LNA) or unlocked nucleic acid (UNA) form can also be used.

In the present invention, the retinal or choroidal disease may be a vascular permeability-related retinal or choroidal disease, but is not limited thereto. The "vascular permeability-related retinal or choroidal disease" is a retinal disease that results from disruption of the normal regulation of vascular permeability, and generally refers to a retinal or choroidal disease causing bleeding, edema and inflammation due to an increase in permeability caused by changes in blood vessels.

In the present invention, the retinal or choroidal disease may be one or more selected from the group consisting of retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular capillarectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration due to glaucoma, and ischemic optic neuropathy, but is not limited thereto.

In the present invention, age-related macular degeneration (AMD) is a disease caused by various changes that occur with aging in the macula of the retina, which plays a very important role in vision. In the present invention, the age-related macular degeneration may include dry (nonexudative) AMD or wet (exudative) AMD. Dry AMD refers to cases in which lesions such as drusen or retinal pigment epithelium atrophy occur in the retina, and accounts for nearly 90% of AMD. As the visual cells present in the macula gradually atrophy, the vision gradually deteriorates over time, and the AMD may develop into a wet form. In wet AMD, choroidal neovascularization grows under the retina, and this neovascularization itself or bleeding, exudation, and the like from the blood vessels is are likely to cause severe visual impairment, and may lead to blindness due to disc-shaped atrophy, severe bleeding, and the like within several months to several years after the onset of the disease.

In the present invention, diabetic retinopathy is one of the microvascular complications that occur in diabetes, and occurs due to the functional morphological changes of capillaries, such as ischemia with increased vascular permeability and neovascularization of the retina due to hyperglycemia and various biochemical changes accompanying the same. In the present invention, the diabetic retinopathy may include non-proliferative or proliferative retinopathy, but is not limited thereto. Non-proliferative retinopathy refers to a condition in which the small blood vessels of the retina weaken to leak serum or become clogged, thereby interrupting nutritional supply. It is known that proliferative retinopathy is caused by the formation of new blood vessels in areas of poor blood circulation, and bleeding occurring from the new blood vessels leads to blindness within 5 years unless appropriate treatment is given.

In the present invention, diabetic chorioretinopathy (diabetic choroidopathy) is one of the microvascular complications that occur in the choroidal blood vessels in diabetes, and occurs as chronic hyperglycemia inhibits fenestration and endothelial transport in the choroidal capillary layer, and the endothelial transport of macromolecules between the choroid and the retina is damaged by diabetes. Furthermore, the loss and degeneration of pericytes are also accompanied by structural and functional changes in large choroidal vessels such as arteries and veins, leading to inflammation and ischemia of the choroidal tissue. The diabetic chorioretinopathy may be a disease in which vision deteriorates due to the induction of structural and functional abnormalities in the choroid, which cause retinal malnutrition and the accumulation of metabolic waste.

In the present invention, the choroid is one of the eyeball walls located between the sclera and the retina, and is a thin membrane rich in blood vessels and melanocytes in the posterior eyeball. It is known that the choroid plays a role in preventing light incident from the outside from being scattered, and acts as the choroid at the posterior part of the eye and the iris at the anterior part of the eye.

In the present invention, the composition may inhibit the proliferation or distribution of smooth muscle cells, but is not limited thereto.

In the present invention, the composition may promote the proliferation or distribution of pericytes, but is not limited thereto.

In the present invention, the composition may reduce drusen, but is not limited thereto.

In the present invention, the composition may inhibit retinal neovascularization or vascular leakage, but is not limited thereto.

In the present invention, the composition may inhibit the dilation or leakage of choroidal vessels, but is not limited thereto.

In the present invention, the composition may further include an anti-VEGF agent, but is not limited thereto. In the present invention, the anti-VEGF agent may be, for example, one or more selected from the group consisting of bevacizumab, ranibizumab, pegaptanib, pazopanib, sunitinib, sorafenib, regorafenib, cabozantinib, lenvatinib, ponatinib, axitinib, tivozanib, ramucirumab, vandetanib, brolucizumab, faricimab and aflibercept, but is not limited thereto. Further, the anti-VEGF agent may include a peptide that binds to vascular epidermal growth factors (VEGFs) to prevent or reduce the binding to the receptors thereof, an antibody that binds to VEGF, a nucleic acid capable of binding to VEGF, and the like, but is not limited thereto.

The composition of the present invention may be administered simultaneously or sequentially with the anti-VEGF agent, but is not limited thereto. In the present invention, the composition including the ACTA2 inhibitor as an active ingredient may be formulated so as to be present in one container in the form of a mixture with an anti-VEGF agent, and may be formulated such that they are present in separate containers and administered simultaneously or sequentially. In addition, the composition including the ACTA2 inhibitor as an active ingredient may be administered as a secondary therapy after treatment with an anti-VEGF agent, but is not limited thereto.

The present invention may also include a pharmaceutically acceptable salt of an ACTA2 inhibitor as an active ingredient. In the present invention, the term "pharmaceutically acceptable salt" includes a salt derived from a pharmaceutically acceptable inorganic acid, organic acid, or base. As used herein, the term "sitologically acceptable salt" includes a salt derived from a sitologically acceptable organic acid, inorganic acid, or base. As used herein, the term "veterinary acceptable salt" includes a salt derived from a veterinary acceptable inorganic acid, organic acid, or base.

Examples of a suitable acid include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and the like. An acid addition salt may be prepared by a typical method, for example, dissolving a compound in an excessive amount of an aqueous acid solution and precipitating the salt using a water-miscible organic solvent such as methanol, ethanol, acetone or acetonitrile. In addition, the acid addition salt may be prepared by heating an equimolar amount of compound and an acid or alcohol in water, and then evaporating the mixture to dry the mixture, or suction-filtering the precipitated salt.

A salt derived from a suitable base may include an alkali metal such as sodium and potassium, an alkaline earth metal such as magnesium, and ammonium and the like, but is not limited thereto. An alkali metal or alkaline earth metal salt may be obtained by, for example, dissolving the compound in an excessive amount of an alkali metal hydroxide or alkaline earth metal hydroxide solution, filtering a non-soluble compound salt, evaporating the filtrate, and drying the resulting product. In this case, it is particularly suitable to prepare a sodium, potassium or calcium salt as the metal salt from the pharmaceutical perspective, and the corresponding silver salt may also be obtained by reacting an alkali metal or alkaline earth metal salt with a suitable silver salt (for example, silver nitrate).

The content of the ACTA2 inhibitor in the composition of the present invention can be appropriately adjusted according to the symptoms of the disease, the degree of progression of the symptoms, the condition of the patient, and the like, and may be, for example, 0.0001 to 99.9 wt%, or 0.001 to 50 wt%, but is not limited thereto. The content ratio is a value based on a dry amount from which the solvent is removed.

The pharmaceutical composition of the present invention may further include an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, an external preparation such as a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be included in the composition according to the present invention include lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, and polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, sucrose, magnesium aluminum silicate, a D-sorbitol solution, and light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

As an additive for liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ether, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In a syrup according to the present invention, a solution of sucrose, other sugars or sweeteners, and the like may be used, and a fragrance, a colorant, a preservative, a stabilizer, a suspending agent, an emulsifier, a thickener, and the like may be used, if necessary.

Purified water may be used for the emulsion according to the present invention, and an emulsifier, a preservative, a stabilizer, a fragrance, and the like may be used, if necessary.

In the suspension according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose (HPMC), HPMC 1828, HPMC 2906, and HPMC 2910 may be used, and a surfactant, a preservative, a stabilizer, a colorant, and a fragrance may be used, if necessary.

The injection according to the present invention may include: a solvent such as distilled water for injection, 0.9% sodium chloride injection, Ringer's injection, dextrose injection, dextrose+sodium chloride injection, PEG, lactated Ringer's injection, ethanol, propylene glycol, non-volatile oil-sesame oil, cottonseed oil, peanut oil, soybean oil, corn oil, ethyl oleate, isopropyl myristate, and benzoic acid benzene; a solubilizing agent such as sodium benzoate, sodium salicylate, sodium acetate, urea, urethane, monoethyl acetamide, butazolidin, propylene glycol, Tweens, nijungtinateamide, hexamine, and dimethylacetamide; a buffer such as a weak acid or a salt thereof (acetic acid and sodium acetate), a weak base and a salt thereof (ammonia and ammonium acetate), an organic compound, a protein, albumin, peptone, and gums; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), and ethylenediaminetetraacetic acid; a sulfating agent such as 0.1% sodium bisulfide, sodium formaldehyde sulfoxylate, thiourea, disodium ethylenediaminetetraacetate, and acetone sodium bisulfite; an analgesic such as benzyl alcohol, chlorobutanol, procaine hydrochloride, glucose, and calcium gluconate; and a suspending agent such as carboxymethyl cellulose sodium, sodium alginate, Tween 80, and aluminum monostearate.

In a suppository according to the present invention, it is possible to use a base such as cacao butter, lanolin, Witepsol, polyethylene glycol, glycerogelatin, methylcellulose, carboxymethyl cellulose, a mixture of stearic acid and oleic acid, Subanal, cottonseed oil, peanut oil, palm oil, cacao butter+cholesterol, lecithin, ranetwax, glycerol monostearate, Tween or Span, Imhausen, monolen (propylene glycol monostearate), glycerin, Adeps solidus, Buytyrum Tego-G, Cebes Pharma 16, hexalide base 95, Cotomar, Hydroxote SP, S-70-XXA, S-70-XX75(S-70-XX95), Hydrokote 25, Hydrokote 711, idropostal, Massa estrarium (A, AS, B, C, D, E, I, T), Massa-MF, Masupol, Masupol-15, Neosupostal-ene, Paramound-B, Suposhiro (OSI, OSIX, A, B, C, D, H, L), suppository base IV types (AB, B, A, BC, BBG, E, BGF, C, D, 299), Supostal (N, Es), Wecobee (W, R, S, M ,Fs), and a tegester triglyceride base (TG-95, MA, 57).

A solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like with an extract. Further, in addition to a simple excipient, lubricants such as magnesium stearate and talc are also used.

A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, a fragrance, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspending agent, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including the type of disease of patients, the severity of disease, the activity of drugs, sensitivity to drugs, administration time, administration route, excretion rate, treatment period, and simultaneously used drugs, and other factors well known in the medical field.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

The pharmaceutical composition of the present invention may be administered to a subject in need via various routes. All methods of administration may be expected, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spray via the mouth or nose, skin administration, transdermal administration, and the like.

The pharmaceutical composition of the present invention is determined by the type of drug that is an active ingredient, as well as various related factors such as the disease to be treated, the route of administration, the age, sex, and body weight of a patient, and the severity of the disease.

As used herein, a "subject" refers to a subject in need of treatment for a disease, and is not limited to vertebrates, but specifically may be applied to a human, a mouse, a rat, a guinea pig, a rabbit, a monkey, a pig, a horse, a cow, a sheep, an antelope, a dog, a cat, a fish and a reptile.

The "administration" as used herein refers to the provision of a predetermined composition of the present invention to a subject in need thereof by any suitable method.

As used herein, the "prevention" refers to all actions that suppress or delay the onset of a target disease, and the "treatment" refers to all actions that ameliorate or beneficially change a target disease and the resulting metabolic abnormalities by administration of the pharmaceutical composition according to the present invention, and the "amelioration" refers to all actions that reduce a target disease and associated parameters, for example, the severity of symptoms, by administration of the composition according to the present invention.

Further, the present invention provides a quasi-drug composition for preventing or treating retinal or choroidal diseases, comprising an ACTA2 inhibitor as an active ingredient.

The quasi-drug composition refers to an article which has a milder action than a drug among articles used for the purpose of diagnosing, treating, ameliorating, alleviating, treating, or preventing a human or animal disease, and for example, according to the Pharmaceutical Affairs Act, the quasi-drug is an article that is not used for pharmaceutical purposes, and includes fiber and rubber products used for the treatment and prevention of human and animal diseases, those that have little or no direct effect on the human body and are similar to those that are not instruments or machines, and bactericidal and pesticides to prevent infectious diseases, and the like.

In the present invention, the quasi-drug composition may be used by being formulated into a dosage form of an ophthalmic composition, and may be used, for example, as one or more formulations selected from the group consisting of an ophthalmic solution, a collyrium, an ophthalmic ointment, an injection, and an eyewash, but is not limited thereto.

Since the present invention may be modified into various forms and include various exemplary embodiments, specific exemplary embodiments will be illustrated in the drawings and described in detail in the Detailed Description. However, the description is not intended to limit the present invention to the specific exemplary embodiments, and it is to be understood that all the changes, equivalents, and substitutions belonging to the spirit and technical scope of the present invention are included in the present invention. When it is determined that the detailed description of the related publicly known art in describing the present invention may obscure the gist of the present invention, the detailed description thereof will be omitted.

Hereinafter, preferred examples for helping with understanding of the present invention will be suggested. However, the following examples are provided only so that the present invention may be more easily understood, and the content of the present invention is not limited by the following examples.

### [Examples]

### Example 1. Experimental Methods

### 1-1. Experimental Material

An siRNA that inhibits ACTA2, which is a major functional gene of smooth muscle cells, was custom made at Bioneer (KR) to inhibit the excessive proliferation and distribution of smooth muscle cells in elderly and diabetic patients. Furthermore, a has-miR-4524a-3p mimic (hereinafter, referred to as miRNA) was purchased from Bioneer (KR). The sequences of the constructed siRNA and purchased miRNA are shown in the following Table 1.

**[Table 1]**

| Name | Sequence | SEQ ID NO |
|---|---|---|
| siRNA #1 sense | CGU ACA CAA GAC UCU CAC A | 3 |
| siRNA #1 antisense | UGU GAG AGU CUU GUG UAC G | 4 |
| siRNA #2 sense | CAC UAU GCA CCU GGA UCA U | 5 |
| siRNA #2 antisense | AUG AUC CAG GUG CAU AGU G | 6 |
| siRNA #3 sense | GAU UUG UUA CUC GUG GUU U | 7 |
| siRNA #3 antisense | AAA CCA CGA GUA ACA AAU C | 8 |
| has-miR-4524a-3p mimic | GAACGAUAGCAGCAUGAACCUGUCUCACUGC AGAAUUAUUUUGAGACAGGCUUAUGCUGCUA UCCUUCA | 9 |

### 1-2. Construction of diabetes-induced animal model

Diabetic chorioretinopathy is a disease which induces the loss of vision because retinal degeneration occurs due to structural and functional changes in choroidal vessels due to long-term diabetes. In diabetic chorioretinopathy, various choroidal vascular changes, such as the dilation of choroidal vessels and the loss of choroidal capillaries, have been reported, and among them, changes in choroidal capillaries are the most important for maintaining the function of the retina. To construct a diabetic chorioretinopathy animal model, first, streptozotocin (STZ, Sigma) was administered intraperitoneally to 7 to 8-week-old male C57BL6/J mice at a dose of 200 mg/kg, and then blood glucose levels were measured one week later to confirm whether hyperglycemia was induced in the mice. Mice in which the blood glucose level was induced to 400 mg/dL or higher were selected and used for subsequent experiments. Eight weeks after the administration of STZ, early symptoms of diabetic chorioretinopathy began to appear.

### 1-3. Construction of age-related macular degeneration (AMD) animal model with decreased pericytes

An age-related macular degeneration model was constructed by selectively removing pericytes expressing PDGF Receptor beta (PDGFRβ) in mice through crossing of PDGFRβ-cre^{ERT2} mice with diphtheria toxin A (ROSA-DTA) mice. Specifically, a PDGFRβ-cre^{ERT2};DTA (PDGFRβ-cre^{ERT2}; DTA^{+/+} and PDGFRβ-cre^{ERT2}; DTA^{+/-} hereinafter, ΔPC) genotype mouse, which is an inducible pericyte specific DTA expressing cell ablation mouse, which is a combination by crossing PDGFRβ-creERT2 corresponding to a Cre-Flox system with a ROSA-DTA mouse, was used. The animal model was constructed using a system in which when a tamoxifen drug is administered to iΔPC, a cyclic recombinase (Cre) activated by a tamoxifen-inducible estrogen receptor (ERT2) is produced, and a DTA gene located behind an FLOX cassette is transcribed as the cassette is linked as a loop and a STOP gene present therebetween is removed.

### 1-4. Construction of laser-induced choroidal neovascularization model

At seven weeks after birth, siRNA or miRNA was injected into the eyeballs of the mice, and one week later, a choroidal neovascularization model mimicking wet macular degeneration was constructed using a laser. After laser irradiation, 2 weeks were set as a time for neovascularization to sufficiently proceed. Thereafter, the eyeballs of the mice were collected and histologically examined.

### 1-5. Electroretinogram (ERG)

The electroretinogram plays an important role in diagnosing and studying abnormalities in retinal function. Under dark adaptation, decreases in the amplitudes of the a-wave and b-wave were observed, the decrease in the amplitude of the a-wave indicates the deterioration in photoreceptor functions, and the decrease in the amplitude of the b-wave indicates the deterioration in the function of bipolar cells. The electroretinogram method was performed with reference to the document [Documenta Ophthalmologica volume 130, pages1-12 (2015)].

### 1-6. Fluorescein angiography (FAG), indocyanine-green angiography and fundus photography

For fluorescein angiography (FAG), indocyanine-green angiography (ICGA) and fundus photography, mice were put under general anesthesia, and then subjected to pupillary dilation using an iridodilator, and then 5 mg/mouse of a fluorescein sodium injection (Alcon) and 0.05 mg/mouse of an indocyanine-green injection (Donindan) were intraperitoneally injected. Wide-angle fundus photographs and wide-angle angiographs were taken using an Optos California System (Optos plc, Scotland, UK).

### 1-7. Whole-mount immunofluorescence and confocal microscopy of retina

Eyeballs of sacrificed mice were harvested and immediately fixed in ice-cold methanol at -20°C for 1 hour or with 4% PFA overnight. The fixed eyes were carefully trimmed to remove the crystalline lens and RPE choroid. Retinal and choroidal tissues were incubated in a blocking solution at room temperature for 1 hour and then incubated at 4°C with each primary antibody diluted in a blocking solution (1:200) overnight. Primary antibodies used are as follows: CD31; BD 550274; Franklin Lakes, NJ, USA, Endomucin; Santacruz SC65495; Dallas, TX, USA, anti-actin ACTA2; Sigma-Aldrich A5228; St. Louis, MO, USA. Samples were washed three times with 0.2% PBST for 5 minutes and incubated with a secondary antibody diluted in 0.1% PBST (1:1000) at 4°C overnight. Thereafter, the samples were washed three times for 5 minutes, mounted with a Prolong Glass Antifade Mountant (Invitrogen P36980; Waltham, MA, USA), and imaged and analyzed under a confocal laser scanning microscope (Leica TCS SP2 and TCSP8X; Wetzlar; Germany). Three-dimensional vessel images and projected images were generated in a Z-series image set using the accompanying software LasX (v.3.6.0).

### 1-8. Image analysis

Morphology measurement and co-localization analysis performed on the retina and choroid were performed as follows. First, for the vessel morphology, the length and area were measured using a LasX program, and then measured values were exported, and for the co-localization analysis, Java-based imaging software (ImageJ, v.1.52p, in the public domain at http://rsb.info.nih.gov/ij; National Institutes of Health (NIH), Bethesda, MD, USA, and FUI) and JaCoP plugin were used. A threshold was set as the most basic value to reduce background sensitivity and applied to all the samples. Manders' coefficient was selected for subject-background selective co-localization evaluation. The coefficient M2 is a coefficient for vascularity versus pericytes.

### 1-9. Statistical analysis

Mouse samples were not randomized during the experiment and were not excluded from the experiment. Further, they were not blinded during the experimental and result analysis. Values are expressed as average ± standard error (SEM). Statistical significance was calculated using the Welchs t-test, Student's t-test, 1-way ANOVA and Tukey's multiple comparison test using PASW Statistics 18 (SPSS v.23). Cases where p < 0.05 was set as statistically significant.

### Example 2. Confirmation of increase in smooth muscle cells (SMCs) in choroidal capillary layer due to aging

### 2-1. Acquisition and preparation process of donated eyeballs

The distribution of smooth muscle cells in aging patients was examined by checking medical records for the presence or absence of underlying diseases in donated eyeballs, and the eyeballs obtained therefrom were used. For classification according to age, only eyeballs of donors without underlying ophthalmic or underlying metabolic diseases were used, and the age was classified into Young (20 years old or older and less than 40 years old) and Old (60 years old or older). In the case of the human donated eyeballs, after the corneal transplantation was completed, the corresponding eyeballs were received, and the sclera and the vitreous body were removed. The retina and the choroid were separated, and then were cut into sections and classified for mRNA analysis samples and for immunofluorescence staining. Tissues for mRNA analysis were put into an RNA later solution (Invitrogen, USA) and stored at -80°C, and tissues for immunofluorescence staining were fixed with 4% PFA overnight.

### 2-2. Whole-mount immunofluorescence and confocal microscopy of donated eyeball tissue

Retinal and choroidal tissues were incubated in a blocking solution at room temperature for 3 hours and then incubated at 4°C with each primary antibody diluted in a blocking solution (1:200) overnight. Primary antibodies used are as follows: CD31 (abcam ab28364; UK) and anti-actin ACTA2 (Sigma-Aldrich A5228; USA). Samples were washed five times with 0.2% PBST for each of 5 minutes and incubated with a secondary antibody diluted in 0.1% PBST (1:1000) at 4°C overnight. Thereafter, the samples were washed five times for each of 5 minutes, mounted with a Prolong Glass Antifade Mountant (Invitrogen P36980; Waltham, MA, USA), and imaged and analyzed under a confocal laser scanning microscope (Leica TCS SP2 and TCSP8X; Wetzlar; Germany). Three-dimensional vessel images and projected images were generated in a Z-series image set using the accompanying software LasX (v.3.6.0).

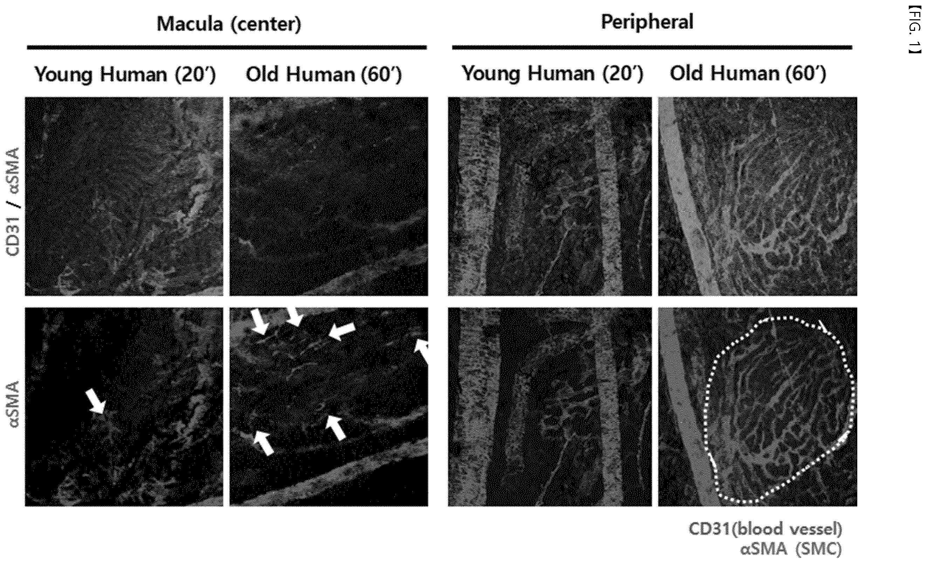

As illustrated in FIG. 1, it was confirmed that the pericytes of the choroidal blood vessels decreased with age, whereas the distribution of smooth muscle cells (SMCs) increased with age.

### 2-3. mRNA expression analysis through next-generation base sequence analysis of donated eyeball tissue

Retinal and choroidal tissues were each included in an RNA later solution, packed in dry ice, and sent to e-biogen (KR), which was commissioned to perform next-generation mRNA base sequence analysis. The analysis was commissioned separately for the tissue of a Young group and the tissue of an Old group, and after the results were received, the ExDEGA program from e-biogen was used to compare the expression of pericyte-related genes in the Young group vs. the Old group, and the expression of pericyte-related genes in the central area vs. the distal area.

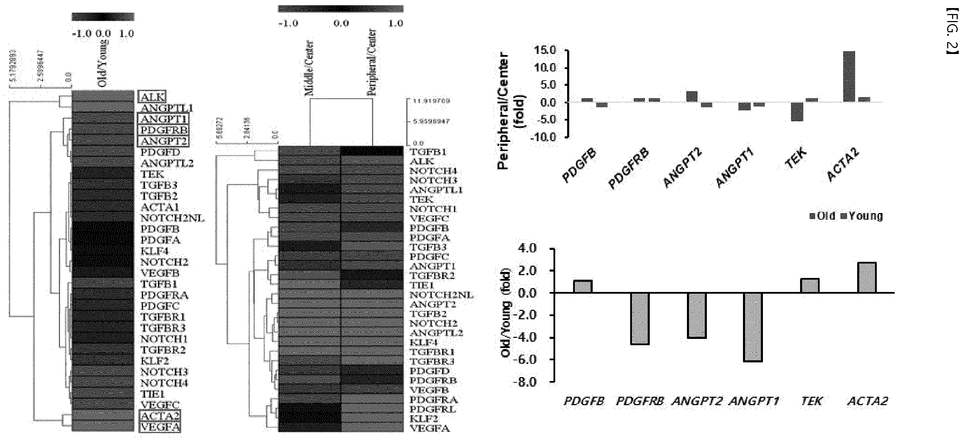

As illustrated in FIG. 2, it was confirmed that platelet derived growth factor receptor β (PDGFRβ, angiopoietin 2 (ANGPT2), and angiopoietin 1 (ANGPT1), which are pericyte-related genes, decreased with age, whereas the smooth muscle cell-related gene actin alpha 2, smooth muscle (ACTA2) increased with age.

Therefore, it was shown that an increase in choroidal capillary smooth muscle cells due to aging interferes with the supply of nutrients from blood vessels to the retina and the excretion of waste from the retina into blood vessels, possibly leading to retinal degeneration.

### Example 3. Confirmation of effect of ACTA2 inhibitor on mouse choroidal tissue

The present inventors confirmed in the document [Int. J. Mol. Sci. 2020, 21, 2158] that smooth muscle cells and their related gene ACTA2 (αSMA) increased in retinal and choroidal capillaries in diabetes-induced experimental animals.

Therefore, in order to inhibit the excessive proliferation and distribution of smooth muscle cells, the present inventors constructed siRNA that inhibits ACTA2, which is a major functional gene of smooth muscle cells, and treated the choroidal tissues of 8-week-old mice with the siRNA, and then, the expression of ACTA2 mRNA was confirmed. For siRNA, #1, #2, #3 alone and #1 to #3 were treated in combination (pooled).

Lyophilized siRNA and miRNA ordered in a volume of 10 nmole were dissolved in 20 µl of tertiary sterilized distilled water and diluted to a concentration of 500 pmole/µl, and then 1 µl of the diluted solution was injected into the vitreous body of the mouse using a 38G INCYTO micro-injection needle (INCYTO, KR) (0.5 nmole/eye). In the case of siRNA, each of #1, #2 and #3 was injected, or #1, #2 and #3 were mixed (pooled) at a molar ratio of 1:1:1 and then injected.

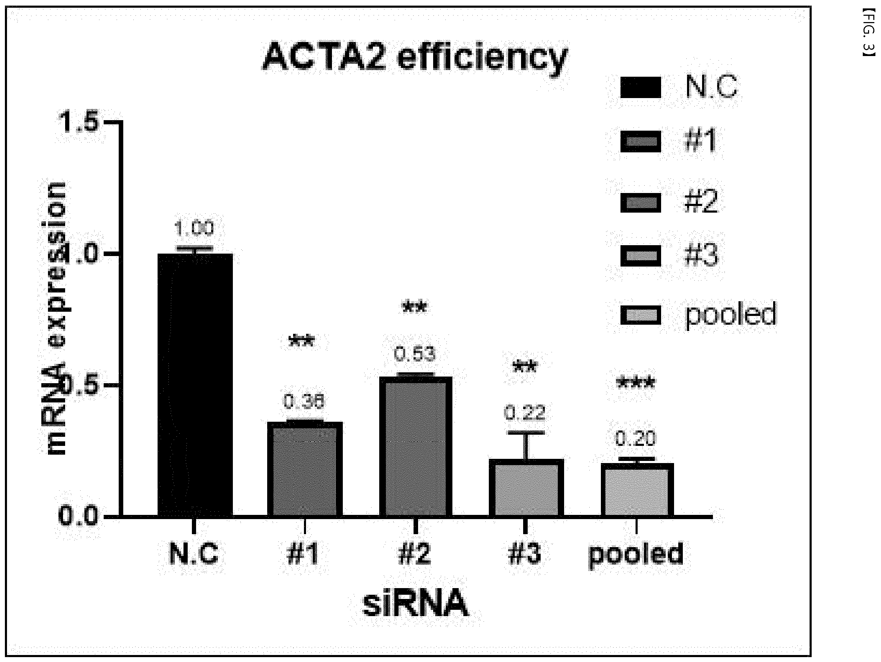

As a result, as illustrated in FIG. 3, it was confirmed that the constructed siRNA significantly suppressed ACTA2 compared to the negative control, and in particular, as a result of combining all of #1, #2, and #3 and treating the siRNA in combination, it could be confirmed that the ACTA2 inhibitory effect was the most excellent

Therefore, #1, #2, and #3 were mixed at a molar ratio of 1:1:1 and used when siRNA was required in the experiments performed below.

### Example 4. Confirmation of effect of ACTA2 siRNA injection into vitreous bodies of aged mice

In order to confirm whether the inhibition of ACTA2 improves choroidal vasculature in aged mice, ACTA2 siRNA and miRNA were injected into the vitreous body. siRNA and miRNA for ACTA2 inhibition were injected into 1.5-year-old aged mice, and one week later, the patterns of smooth muscle cells and pericytes in the retina and choroid were analyzed by whole-mount immunofluorescence staining. Tertiary sterilized distilled water was used as a vehicle, and Control (CTL) is a control in which sterilized distilled water is mixed with scramble siRNA.

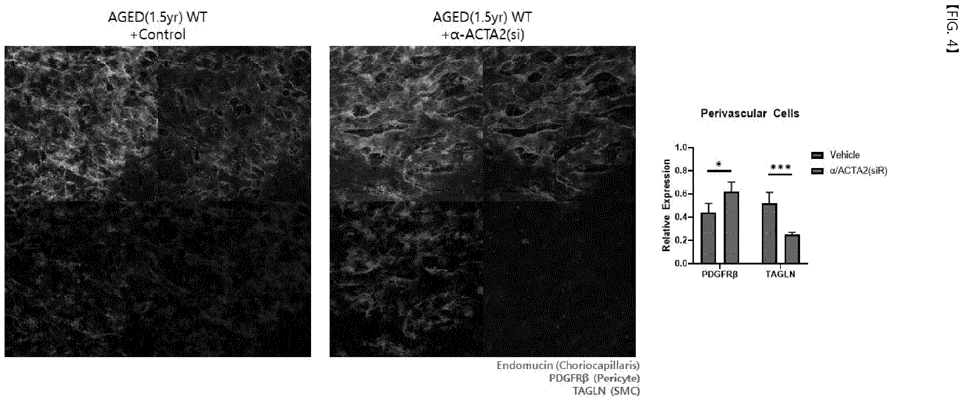

As a result, as illustrated in FIG. 4, it was confirmed that, by siRNA treatment, the number of cells expressing a pericyte marker gene PDGFRβ, which had decreased due to aging, was significantly increased, and the number of cells expressing a smooth muscle cell marker TAGLN significantly decreased.

This indicates that, by administering siRNA, the excessive proliferation and distribution of smooth muscle cells in the choroidal vessels were inhibited, and the distribution of pericytes was increased.

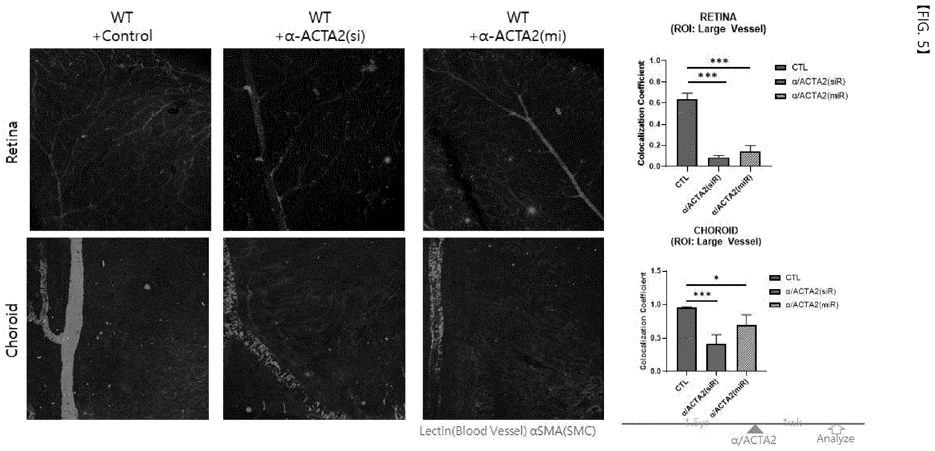

Furthermore, as illustrated in FIG. 5, it was confirmed that as siRNA and miRNA were administered, the number of smooth muscle cells in both the retina and choroid of aged mice was significantly decreased.

The above results show that the ACTA2 inhibitor may be used to treat retinal or choroidal diseases caused by aging.

### Example 5. Confirmation of drusen production inhibitory effect of ACTA2 inhibitor on age-related macular degeneration (AMD)

The effect of the ACTA2 inhibitor was confirmed using the AMD model constructed in Examples 1-3. Two weeks after AMD induction, each of ACTA2 inhibitor siRNA and miRNA were injected intravitreally, and the generation of drusen was analyzed by fundus photography 4 weeks later. Control (CTL) is a control in which sterilized distilled water is mixed with scramble siRNA.

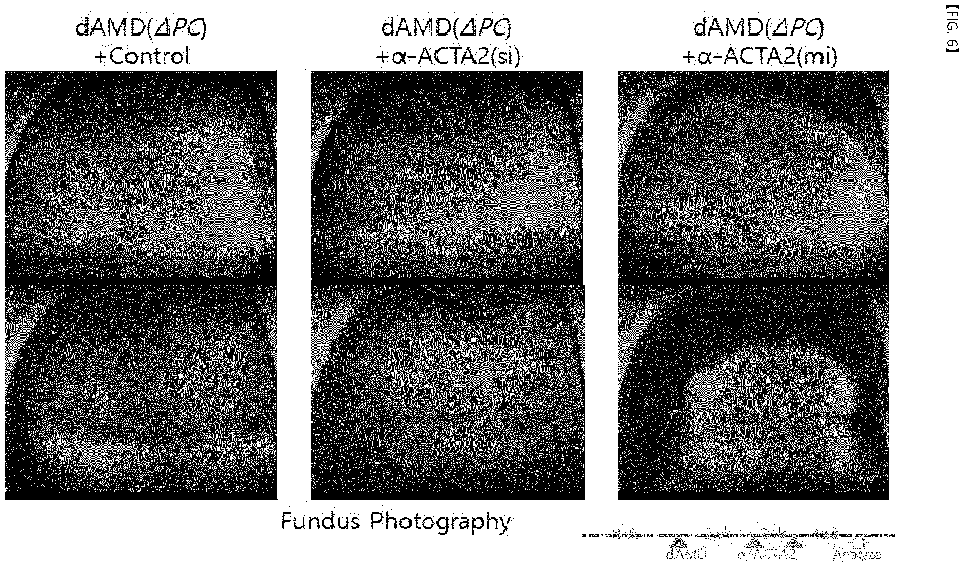

As illustrated in FIG. 6, it could be confirmed that the generation of drusen was effectively inhibited in an experimental group into which the ACTA2 inhibitor was injected compared to the control.

Therefore, it was confirmed that AMD could be treated by inhibiting ACTA2.

### Example 6. Confirmation of retinal function restoration effect of ACTA2 inhibitor on age-related macular degeneration (AMD)

The effect of the ACTA2 inhibitor was confirmed using the AMD model constructed in Examples 1-3. Two weeks after induction of AMD, an ACTA2 inhibitor siRNA and a control vehicle (tertiary sterilized distilled water + scramble siRNA) were each injected into the vitreous body, and visual function was measured by performing an electroretinogram two weeks later.

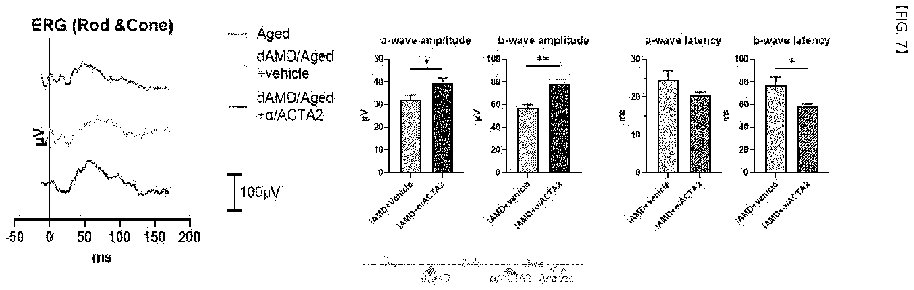

As illustrated in FIG. 7, it was confirmed that as an ACTA2 inhibitor was injected, the electroretinogram waveform was restored similarly to that of normal mice, an a-wave amplitude, which indicates the functional restoration of visual cells, was also increased, a b-wave amplitude, which indicates the restoration of retinal vessels, was also significantly increased. Furthermore, latency was also decreased, indicating that the functions of horizontal and bipolar cells were also restored.

### Example 7. Confirmation of antiangiogenic effect of ACTA2 inhibitor on wet AMD

Using the laser-induced choroidal neovascularization model constructed in Example 1-4, an antiangiogenic effect on wet AMD was confirmed. 1 µl of each ACTA2 inhibitor (siRNA and miRNA) diluted to a concentration of 500 pmole/µl was injected into the vitreous body of the mouse eyeball in 7-week-old mice using a 38G INCYTO micro injection needle (INCYTO, KR) (0.5 nmole/eye). Wet AMD was induced using a laser one week after administration. After blood vessels, pericytes, and smooth muscle cells in 10-week-old mice were stained using immunofluorescence staining, choroidal neovascularization was analyzed by imaging with a confocal microscope. Images acquired with a confocal microscope were analyzed for the area of neovascularization using the above 1-8 method, and the degrees of neovascularization coverage of pericytes and smooth muscle cells were compared through co-localization analysis. Control (CTL) is a control in which sterilized distilled water is mixed with scramble siRNA.

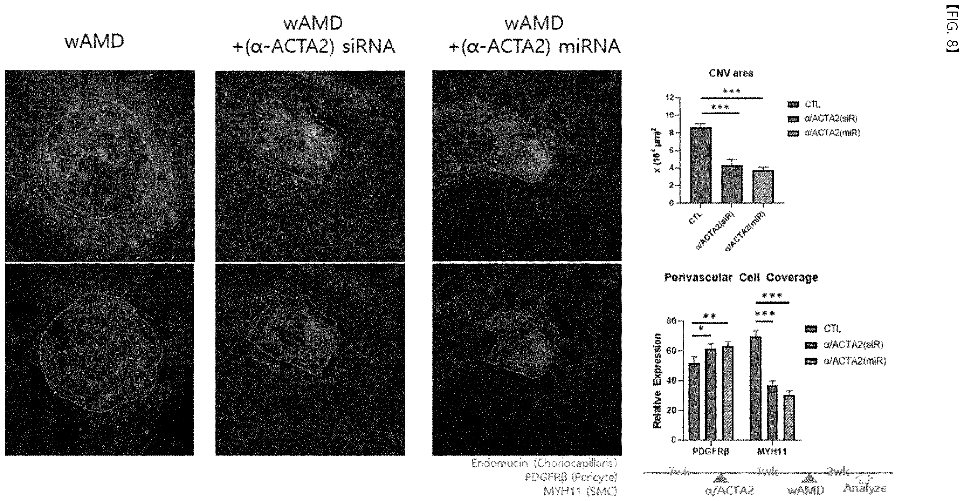

As illustrated in FIG. 8, it could be confirmed that both siRNA and miRNA caused a very significant decrease in the size of choroidal neovascularization (CNV) compared to the control group, and accordingly, the distribution of smooth muscle cells in the corresponding region in the ROI of CNV was significantly reduced compared to the CNV size. In addition, it could be confirmed that PDGFRβ, which is a distribution marker of pericytes, was significantly increased.

### Example 8. Confirmation of therapeutic effect of ACTA2 inhibitor on diabetic mouse model

### 8-1. Confirmation of neovascularization and vascular leakage reduction effects

Using the diabetic mouse model constructed in Example 1-2, the effect of inhibiting abnormal neovascularization and vascular leakage in diabetic retinopathy and diabetic chorioretinopathy models was confirmed. 1 µl of each ACTA2 inhibitor diluted to a concentration of 500 pmole/µl was injected into the vitreous body of the mouse eyeball in 16-week-old mice using a 38G INCYTO micro injection needle (INCYTO, KR) (0.5 nmole/eye). Fluorescein angiography (FAG), fundus photography and indocyanine-green angiography (ICGA) were performed 3 weeks after administration. Control (CTL) is a control in which sterilized distilled water is mixed with scramble siRNA.

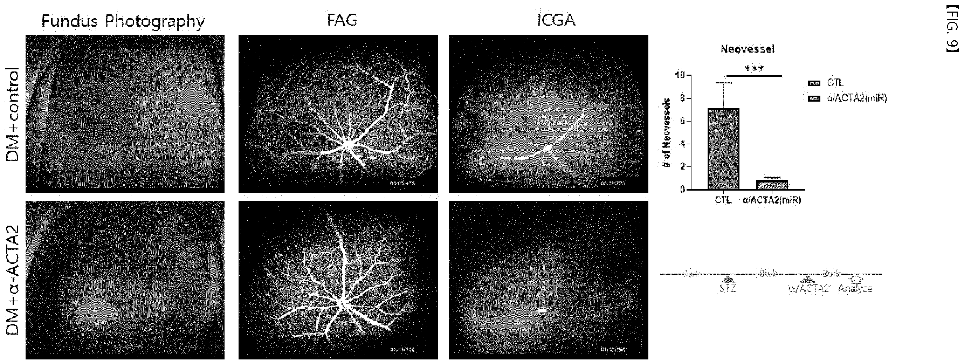

As illustrated in FIG. 9, it was confirmed that as a result of administering an ACTA2 inhibitor, the abnormal neovascularization and vascular leakage of the retina were significantly reduced compared to the control. It was confirmed that an abnormal hyperfluorescence reaction, which indicated choroidal leakage on ICGA, was also reduced by administering the ACTA2 inhibitor.

### 8-2. Confirmation of effect of reducing production of microaneurysm in retina

Three weeks after administration of the ACTA2 inhibitor (siRNA and miRNA) to 16-week-old mice, the production of retinal microvasculature was observed by performing fluorescein angiography. The ACTA2 inhibitor was administered in the same manner as in 8-1.

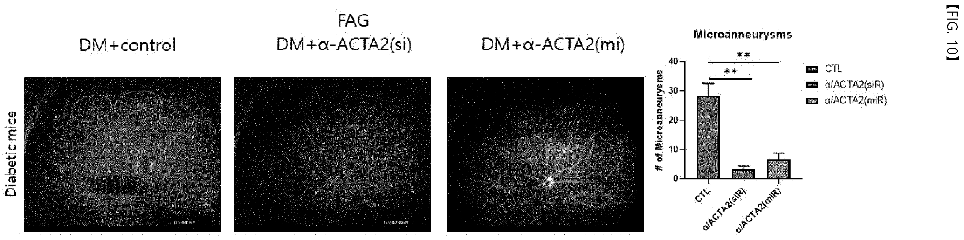

As illustrated in FIG. 10, it was confirmed that as a result of administering the ACTA2 inhibitor to a diabetic mouse model, the retinal microvasculature, which corresponds to a representative vascular change in diabetic retinopathy, was significantly reduced.

### 8-3. Confirmation of dilation and leakage reduction effect of choroidal vessels

Three weeks after administration of the ACTA2 inhibitor (siRNA and miRNA) to 16-week-old mice, the morphology of choroidal vessels was observed by performing indocyanine-green angiography (ICGA). The ACTA2 inhibitor was administered in the same manner as in 8-1.

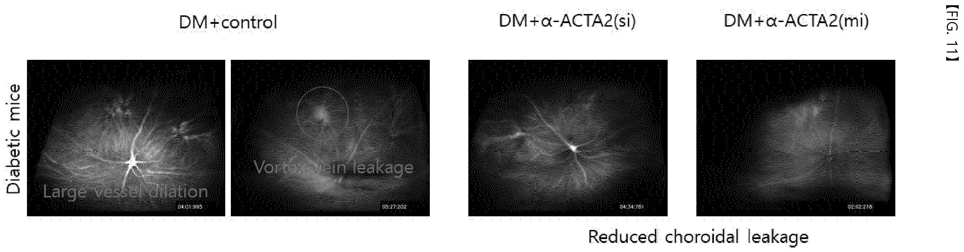

As illustrated in FIG. 11, it was confirmed that as a result of administering the ACTA2 inhibitor to a diabetic mouse model, the dilation and leakage of choroidal vessels, which are representative symptoms of diabetic chorioretinopathy, were significantly reduced.

### 8-4. Confirmation of choroidal inflammation alleviation effect

Three weeks after administration of the ACTA2 inhibitor (siRNA and miRNA) to 16-week-old mice, a choroidal inflammation alleviation effect was confirmed by performing immunofluorescence staining. The ACTA2 inhibitor was administered in the same manner as in 8-1.

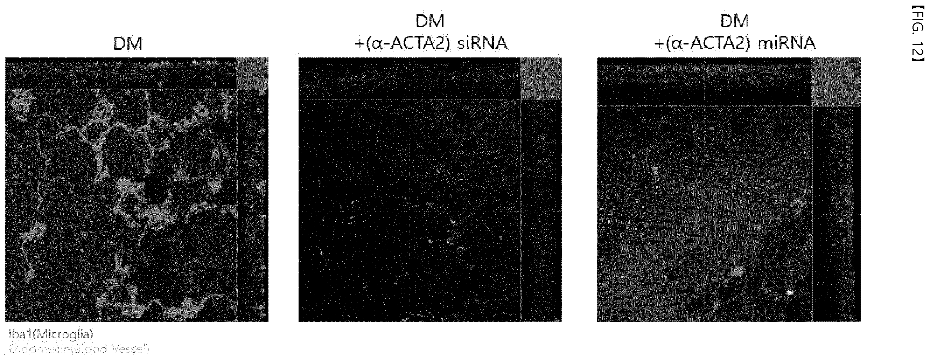

As illustrated in FIG. 12, as a result of administering the ACTA2 inhibitor to a diabetic mouse model, aggregated microglial cells (microglia) in the retinal pigment epithelium and choroidal tissue, were reduced, which is a result supporting that choroidal inflammation, which is a characteristic of diabetic chorioretinopathy, is ameliorated.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described examples are only illustrative in all aspects and not restrictive.

### [Industrial Applicability]

The present inventors have confirmed that, based on the fact an increase in ACTA2 induces a decrease in pericytes and induces the proliferation and distribution of smooth muscle cells, the proliferation and distribution of vascular smooth cells are inhibited, the distribution of pericytes is increased, and the dilation and leakage of choroid vessels are inhibited, as a result of administration of ACTA2 inhibitor siRNA and miRNA to an animal model having a retinal or choroidal disease. Therefore, the ACTA2 inhibitor can be usefully used for the treatment of a retinal or choroidal disease, and thus has industrial applicability.

## Claims

1. A pharmaceutical composition for preventing or treating a retinal or choroidal disease, comprising an actin alpha 2, smooth muscle (ACTA2) inhibitor as an active ingredient.

2. The pharmaceutical composition of claim 1, wherein the inhibitor is an ACTA2 activity inhibitor or an expression inhibitor.

3. The pharmaceutical composition of claim 2, wherein the activity inhibitor is one or more selected from the group consisting of a compound, a peptide, a peptidomimetic, a substrate analogue, an aptamer and an antibody, which specifically bind to an ACTA2 protein.

4. The pharmaceutical composition of claim 2, wherein the expression inhibitor is one or more selected from the group consisting of an antisense nucleotide, RNAi, siRNA, miRNA, shRNA, and a ribozyme, which complementarily bind to the mRNA of an ACTA2 gene.

5. The pharmaceutical composition of claim 4, wherein the siRNA comprises one or more base sequences selected from the group consisting of SEQ ID NOS: 3 to 8.

6. The pharmaceutical composition of claim 5, wherein the siRNA comprises one or more siRNAs selected from the group consisting of SEQ ID NOS: 3 and 4; SEQ ID NOS 5 and 6; and SEQ ID NOS: 7 and 8.

7. The pharmaceutical composition of claim 4, wherein the miRNA is miR-4524a.

8. The pharmaceutical composition of claim 1, wherein the retinal or choroidal disease is one or more selected from the group consisting of retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular capillarectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration due to glaucoma, and ischemic optic neuropathy.

9. The pharmaceutical composition of claim 3, wherein the ACTA2 protein comprises an amino acid sequence represented by SEQ ID NO: 1.

10. The pharmaceutical composition of claim 1, wherein the composition is administered by one or more routes selected from the group consisting of oral, subcutaneous, intraperitoneal, intrapulmonary, intranasal, intramuscular, intravenous, intraarterial and topical ocular administration.

11. The pharmaceutical composition of claim 10, wherein the topical ocular administration is one selected from the group consisting of intraconjunctival administration, intravitreal administration, subretinal administration, suprachoroidal administration, subconjunctival administration, and sub-Tenon's capsule administration.

12. The pharmaceutical composition of claim 1, wherein the composition further comprises an anti-VEGF agent.

13. The pharmaceutical composition of claim 12, wherein the composition defined in claim 1 is administered simultaneously or sequentially with the anti-VEGF agent.

14. The pharmaceutical composition of claim 1, wherein the composition has one or more effects selected from the group consisting of the following items:
inhibition of the proliferation or distribution of smooth muscle cells;
promotion of the proliferation or distribution of pericytes;
drusen reduction;
inhibition of retinal neovascularization or vascular leakage; and
inhibition of the dilation or leakage of choroidal vessels.

15. A quasi-drug composition for preventing or treating a retinal or choroidal disease, comprising an ACTA2 inhibitor as an active ingredient.

16. A method for preventing, ameliorating, or treating a retinal or choroidal disease, the method comprising administering a composition comprising an ACTA2 inhibitor as an active ingredient to a subject in need.

17. A use of a composition comprising an ACTA2 inhibitor as an active ingredient for preventing, ameliorating, or treating a retinal or choroidal disease.

18. A use of an ACTA2 inhibitor for producing a drug for preventing or treating a retinal or choroidal disease.
